# EUROPÄISCHE PATENTSCHRIFT

(11) **EP 2 754 493 B1**
(45) Veröffentlichungstag und Bekanntmachung des Hinweises auf die Patenterteilung: **08.05.2019**
(21) Anmeldenummer: 13197916.3
(22) Anmeldetag: 18.12.2013
(51) Int. Cl.: B01L 3/00, A61B 10/00, G01N 1/00, G01N 1/02

(54) **KAMMER ZUR AUFNAHME EINER PROBE MIT INTEGRIERTER SCHNEIDE**
CHAMBER FOR RECEIVING A SAMPLE WITH INTEGRATED CUTTING DEVICE
CHAMBRE DESTINÉE À CONTENIR UN ÉCHANTILLON AVEC UNE LAME INTÉGRÉE

(30) Priorität: 14.01.2013 DE 102013200357
(43) Veröffentlichungstag der Anmeldung: 16.07.2014
(73) Patentinhaber: ROBERT BOSCH GMBH, 70442 Stuttgart (DE)
(72) Erfinder: Sodan, Lars, 72764 Reutlingen (DE); Behrens, Holger, 70192 Stuttgart (DE); Zinober, Sven, 71292 Friolzheim (DE); Seidl, Karsten, 70839 Gerlingen (DE)

(56) Entgegenhaltungen:
- WO-A2-2009/034563
- WO-A2-2012/018741
- GB-A- 2 432 420
- US-A1- 2009 030 342

## Beschreibung

### Stand der Technik

Lab-on-a-chip Systeme sind mikrofluidische Vorrichtungen, welche mehrere Funktionalitäten eines makroskopischen Labors in einem etwa plastikkartengroßen Kunststoffsubstrat vereinigen und insbesondere in der medizinischen Anwendung zur Untersuchung von Patientenproben wie beispielsweise Blut, Urin oder Sputum Anwendung finden. Die Patientenprobe wird dabei üblicherweise mittels eines Probenträgers in eine Kammer zur Aufnahme der Probe eingeführt.

Aus der WO 2006/121997 und der WO 2008/140568 sind Systeme bekannt, bei denen die Probe in flüssiger Form durch Spritzen eingebracht wird. In der WO 2010/127464 ist ein System beschrieben, bei welchem ein Probenträger in eine Kammer eingeführt und ein Teil des Probenträgers durch einen beweglichen Deckel von einem Rest des Probenträgers abgetrennt wird.

Aus dem Dokument GB 2 432 420 A ist eine Vorrichtung zur Extrahierung einer Probe von einem Probenträger mit einer Karität zur Aufnahme des Probenträgers bekannt. Dabei wird der mit der Probe behaftete Teil des Probenträgers in die Kammer eingefürht und vom Rest des Probenträgers abgetrennt und anschließend die Kammer verschlossen und der aufgenommene Teil des Probenträgers mit einer Flüssigkeit, beispielsweise einer Waschflüssigkeit umspült.

### Offenbarung der Erfindung

### Vorteile der Erfindung

Die erfindungsgemäße Vorrichtung mit den Merkmalen des unabhängigen Anspruchs 1 sowie das erfindungsgemäße Verfahren des nebengeordneten Anspruchs 5 weisen demgegenüber den Vorteil auf, dass die Kammer zur Aufnahme mindestens einer Probe im Bereich der Öffnung einen Vorsprung aufweist, welcher dazu ausgelegt ist, bei einer Krafteinwirkung des Vorsprungs auf einen sich in die Kammer erstreckenden Teils des aufgenommenen Probenträgers einen Teil des Probenträgers von einem Rest des Probenträgers abzutrennen. Der abgetrennte Teil des Probenträgers, welcher die zu untersuchende Patientenprobe umfasst, verbleibt somit in der Kammer, während der nicht weiter benötigte Rest des Probenträgers vorzugsweise sicher entsorgt werden kann. Erfindungsgemäß wird somit keine Relativbewegung von Bauteilen des mikrofluidischen Chips zur Abtrennung des die Probe tragenden Teils des Probenträgers benötigt.

Durch die in den abhängigen Ansprüchen aufgeführten Maßnahmen sind vorteilhafte Weiterbildungen und Verbesserungen der im unabhängigen Anspruch beschriebenen Vorrichtung möglich.

Erfindungsgemäß weist der Vorsprung eine Kante auf, die bei einer Krafteinwirkung des Vorsprungs auf den sich in die Kammer erstreckenden Teil des aufgenommenen Probenträgers die Wirkung einer Schneide entfaltet. Besonders vorteilhaft ist hierbei, dass der Vorsprung die Form einer sich verjüngenden Kante aufweist, da dadurch die notwendige Kraft zur Abtrennung des Teils des Probenträgers vom Rest des Probenträgers deutlich geringer ausfällt.

Erfindungsgemäß weist die Kammer mindestens einen weiteren Vorsprung auf, welcher zur Fixierung des aufgenommenen Probenträgers ausgelegt ist. Vorteilhafterweise reduziert oder verhindert der mindestens eine weitere Vorsprung eine laterale Bewegung des aufgenommenen Probenträgers und unterstützt somit die Abtrennung eines Teils des Probenträgers vom Rest des Probenträgers bei einer Krafteinwirkung des Vorsprungs auf den Probenträger.

In der Erfindung ist mindestens einer der Vorsprünge einstückig mit mindestens einem Teil der Kammer ausgeformt. Dies ermöglicht eine vereinfachte Herstellung der Kammer.

Vorzugsweise ist die Öffnung der Kammer verschließbar. Dies hat den Vorteil, dass die aufgenommene Probe auch bei einer beliebigen Bewegung der Kammer sicher in der Kammer verbleibt und dass bei einem vorzugsweisen Hinzufügen einer Flüssigkeit zur Prozessierung der Probe diese Flüssigkeit ebenfalls nicht aus der Kammer austreten kann.

In einer vorteilhaften Weiterbildung ist die Kammer zur Aufnahme einer Probe Teil einer Einrichtung zum Lysieren von Zellen, insbesondere zum Lysieren mittels Ultraschall. Vorzugsweise findet die Lyse der Zellen nach Aufnahme des Probenträgers und Abtrennung des Teils des Probenträgers vom Rest des Probenträgers in derselben Kammer statt. Dabei kann die Probe direkt mit Ultraschall beaufschlagt werden oder auch vorher mit einem geeigneten Fluid in Kontakt gebracht werden, welches vorzugsweise die Lyse der Zellen erleichtert.

In einer besonders vorteilhaften Ausgestaltung der Erfindung ist die Kammer Teil einer Vorrichtung zur Analyse einer Probe, insbesondere einer organischen Substanz. Die Vorrichtung ist vorzugsweise als Teil eines mikrofluidischen Labors, einem sogenannten "lab-on-a-chip", ausgestaltet.

Gegenstand der Erfindung ist auch ein Verfahren zur Aufnahme von mindestens einer Probe, insbesondere einer organischen Substanz, in eine Kammer, welches in einem ersten Schritt ein Einbringen eines vorzugsweise stabförmigen Probenträgers in die Kammer umfasst. In einem zweiten Schritt wird der Probenträger gegen einen Vorsprung in der Kammer bewegt, um eine Krafteinwirkung des Vorsprungs auf den sich in die Kammer erstreckenden Teil des aufgenommenen Probenträgers auszuüben, so dass ein Teil des Probenträgers von einem Rest des Probenträgers abgetrennt wird. In einer vorteilhaften Weiterbildung wird in einem dritten Schritt die Öffnung der Kammer verschlossen.

### Kurze Beschreibung der Zeichnungen

Ausführungsbeispiele der Erfindung sind in den Zeichnungen dargestellt und in der nachfolgenden Beschreibung näher erläutert. Es zeigen:
- Figur 1: eine erfindungsgemäße Kammer zur Aufnahme mindestens einer Probe sowie einen stabförmigen Probenträger und einen Deckel zum Verschließen der Öffnung der Kammer,
- Figur 2: eine Öffnung der erfindungsgemäßen Kammer,
- Figur 3: eine weitere Ausführungsform der erfindungsgemäßen Kammer sowie einen Teil des Probenträgers und eine Kappe zum Verschließen der Öffnung der Kammer,
- Figur 4: ein Flussdiagramm des erfindungsgemäßen Verfahrens.

### Ausführungsformen der Erfindung

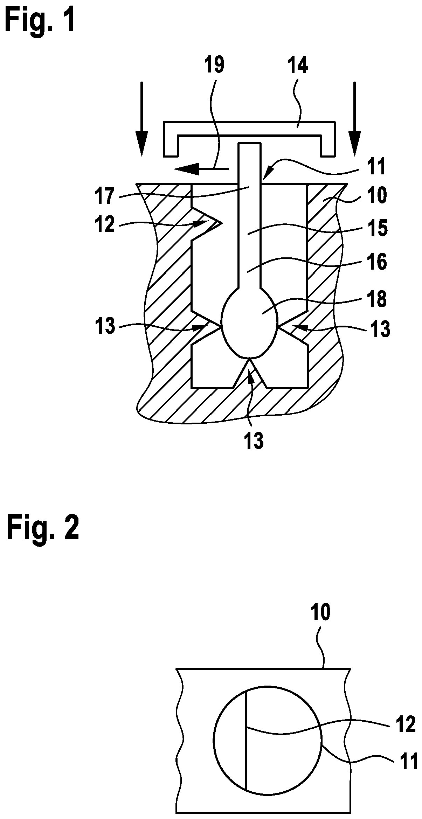

In der Figur 1 ist eine erste Ausführungsform der Erfindung dargestellt. Die Kammer 10 weist an ihrer Oberseite eine Öffnung 11 sowie in dem Bereich der Öffnung einen ersten Vorsprung 12 auf, welcher vorteilhafterweise in Form einer sich verjüngenden Kante ausgebildet ist. Ein zumindest teilweise in der Kammer 10 aufgenommener Probenträger 15, welcher in diesem Ausführungsbeispiel als sogenannter swab in Form eines Stäbchens mit einem als Wattebausch ausgelegten Ende ausgebildet ist, wird im unteren Bereich der Kammer durch weitere Vorsprünge 13 in vorteilhafter Weise fixiert. Die Fixierung erfolgt dabei vorzugsweise an dem Wattebausch des Probenträgers 15, welcher die Probe beinhaltet, und reduziert somit die Bewegungsfreiheit der Probe. Der erste Vorsprung 12 ist dazu ausgelegt, bei einer Krafteinwirkung des ersten Vorsprungs 12 auf den sich in die Kammer 10 erstreckenden Teil des aufgenommenen Probenträgers 15, welche vorzugsweise durch eine Bewegung des Probenträgers 15 in Richtung 19 des ersten Vorsprungs 12 verursacht wird, einen Teil des Probenträgers 16, welcher den die zu untersuchende Probe beinhaltenden Wattebausch umfasst, von einem Rest des Probenträgers 17 abzutrennen. Vorzugsweise sind der erste Vorsprung 12 und/oder die weiteren Vorsprünge 13 einstückig mit mindestens einem Teil der Kammer 10 ausgeformt, was den Herstellungsprozess der Kammer 10, beispielsweise durch ein Spritzgussverfahren, vereinfacht. Die Kammer 10, der erste Vorsprung 12 und/oder die weiteren Vorsprünge 13 können beispielsweise aus Kunststoff, Metall oder Keramik gefertigt sein.

In einer vorteilhaften Weiterbildung der Erfindung wird die Öffnung 11 der Kammer 10 mit einem geeigneten Deckel 14 verschlossen. Der Deckel 14 kann in Form einer Kappe ausgebildet sein, welche beispielsweise auf die Öffnung 11 der Kammer 10 aufgeschraubt oder durch einen Klickverschluss mit der Kammer 10 verbunden wird.

In einer besonders vorteilhaften Weiterbildung der Erfindung stellt die Kammer 10 Teil eine Einrichtung zum Lysieren von Zellen dar. Die Lyse der Zellen erfolgt vorzugsweise mittels Ultraschall und/oder einer lysierenden chemischen Substanz, beispielsweise einem chaotropen Salz, und/oder enzymatischen Stoffen wie beispielsweise Proteasen oder Lysozym. Der in der Kammer 10 verbleibende Teil 16 des Probenträgers wird vorzugsweise mit einer Flüssigkeit umspült, welche die in dem Wattebausch 18 des Probenträgers 15 enthaltene Probe herauslöst und/oder zumindest einen Teil der sich in der Probe befindlichen Zellen lysiert.

Figur 2 zeigt eine Draufsicht auf die Öffnung 11 der erfindungsgemäßen Kammer 10 in einer weiteren Ausführungsform. Die Öffnung 11 der Kammer 10weist hier eine kreisartige Form auf, kann aber auch in Form eines Rechtecks, eines Quadrats oder einer Ellipse ausgebildet sein. Der Vorsprung 12 deckt in dieser Ausführungsform einen Teil der Öffnung 11 ab.

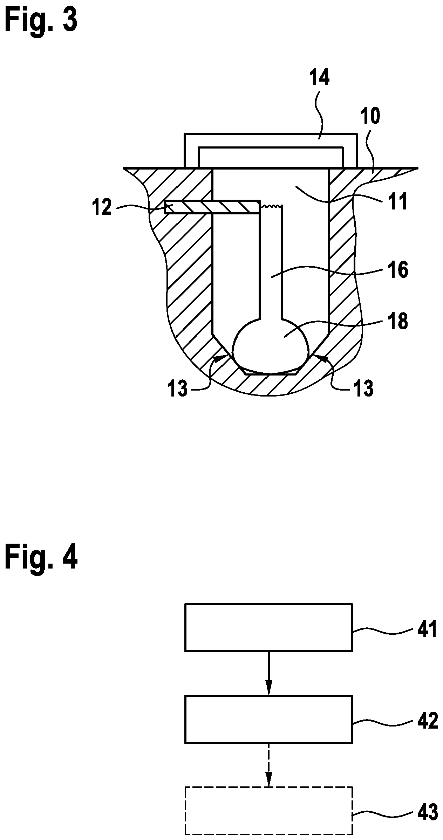

Figur 3 zeigt eine Ausführungsform der erfindungsgemäßen Kammer 10, wobei die Öffnung 11 der Kammer 10 durch einen Deckel 14 verschlossen ist. Ein Teil 16 des in die Kammer 10 aufgenommenen Probenträgers 15 wurde von einem Rest 17 des Probenträgers 16 mittels Krafteinwirkung des Vorsprungs 12 abgetrennt, wobei in diesem Ausführungsbeispiel der Vorsprung 12 zumindest teilweise aus Kunststoff, aus Metall oder aus Keramik besteht und vorzugsweise mit der Kammer 10 durch ein Spritzgussverfahren verbunden ist.

Figur 4 zeigt ein Flussdiagramm für ein Ausführungsbeispiel des erfindungsgemäßen Verfahrens. In einem ersten Schritt 41 wird der Probenträger 15 in die Kammer 10 eingebracht und in einem zweiten Schritt 42 durch Bewegung des Probenträgers 15 gegen einen Vorsprung 12 eine Krafteinwirkung des Vorsprungs 12 auf den sich in die Kammer 10 erstreckenden Teil des Probenträgers 15 ausgeübt, so dass ein Teil 16, welcher die zu untersuchende Probe vorzugsweise in einem Wattebausch umfasst, des Probenträgers 15 von einem Rest 17 des Probenträgers 15 abgetrennt wird. Vorzugsweise wird in einem weiteren Schritt 43 die Öffnung 11 der Kammer 10 durch einen geeigneten Deckel 14 verschlossen. Im Anschluss kann wie oben beschrieben vorzugsweise die Lyse von in der Probe enthaltenen Zellen in derselben Kammer 10 stattfinden oder die Probe mit einer geeigneten Flüssigkeit aus dem abgetrennten Teil 16 des Probenträgers 15 zur weiteren Analyse herausgelöst werden.

## Patentansprüche

1. Kammer (10) zur Aufnahme von mindestens einer Probe, insbesondere einer organischen Substanz, umfassend eine Öffnung (11) an einer Oberseite der Kammer (10) zur Aufnahme eines vorzugsweise stabförmigen Probenträgers (15), wobei die Kammer (10) im Bereich der Öffnung (11) einen Vorsprung (12) aufweist, wobei der Vorsprung (12) durch eine Form einer sich verjüngenden Kante dazu ausgelegt ist, bei einer Krafteinwirkung des Vorsprungs (12) auf einen sich in die Kammer (10) erstreckenden Teil des aufgenommenen Probenträgers (15) einen Teil (16) des Probenträgers von einem Rest (17) des Probenträgers (15) abzutrennen, **dadurch gekennzeichnet, dass** die Kammer (10) in einem bezüglich der Oberseite unteren Bereich der Kammer (10) mindestens einen weiteren Vorsprung (13) aufweist, welcher zur Fixierung des aufgenommenen Probenträgers (15) ausgelegt ist, und wobei mindestens einer der Vorsprünge (12, 13) einstückig mit mindestens einem Teil der Kammer (10) ausgeformt ist.

2. Kammer (10) nach einem der vorangegangenen Ansprüche, **dadurch gekennzeichnet, dass** die Öffnung (11) der Kammer (10) verschließbar ist.

3. Kammer (10) nach einem der vorangegangenen Ansprüche, **dadurch gekennzeichnet, dass** die Kammer (10) Teil einer Einrichtung zum Lysieren von Zellen, insbesondere zum Lysieren mittels Ultraschall, ist.

4. Vorrichtung zur Analyse mindestens einer Probe, insbesondere einer organischen Substanz, aufweisend eine Kammer (10) nach einem der vorangegangenen Ansprüche.

5. Verfahren zur Aufnahme von mindestens einer Probe, insbesondere einer organischen Substanz, in eine Kammer (10), wobei die Kammer (10) im Bereich einer an einer Oberseite der Kammer (10) befindlichen Öffnung (11) der Kammer einen Vorsprung (12) umfasst und wobei der Vorsprung (12) eine Form einer sich verjüngenden Kante aufweist, die folgenden Schritte umfassend:
a) Einbringen eines vorzugsweise stabförmigen Probenträgers (15) in die Kammer (10) und Fixierung des Probenträgers (15) durch mindestens einen weiteren in einem bezüglich der Oberseite unteren Bereich der Kammer (10) befindlichen Vorsprung (13), wobei mindestens einer der Vorsprünge (12, 13) einstückig mit mindestens einem Teil der Kammer (10) ausgeformt ist.
b) Bewegen des Probenträgers (15) gegen den Vorsprung (12) in der Kammer (10) zur Ausübung einer Krafteinwirkung des Vorsprungs (12) auf den sich in die Kammer (10) erstreckenden Teil des aufgenommenen Probenträgers (15), so dass ein Teil (16) des Probenträgers von einem Rest (17) des Probenträgers (15) abgetrennt wird.

6. Verfahren nach Anspruch 6, wobei nach den Schritten a und b folgender Schritt erfolgt:
d) Verschließen der Öffnung (11) der Kammer (10).

## Claims

1. Chamber (10) for receiving at least one sample, in particular an organic substance, comprising an opening (11) on an upper side of the chamber (10) for receiving a preferably bar-shaped sample carrier (15), wherein the chamber (10) has a projection (12) in the region of the opening (11), wherein, by a form of a tapering edge, the projection (12) is designed for detaching a part (16) of the sample carrier from a remainder (17) of the sample carrier (15) when a force applied by the projection (12) acts on the part of the received sample carrier (15) that extends into the chamber (10), **characterized in that** the chamber (10) has in a lower region of the chamber (10) with respect to the upper side at least one further projection (13), which is designed for fixing the received sample carrier (15), and wherein at least one of the projections (12, 13) is formed in one piece with at least part of the chamber (10).

2. Chamber (10) according to one of the preceding claims, **characterized in that** the opening (11) of the chamber (10) is closable.

3. Chamber (10) according to one of the preceding claims, **characterized in that** the chamber (10) is part of a device for lysing cells, in particular for lysing by means of ultrasound.

4. Apparatus for analysing at least one sample, in particular an organic substance, having a chamber (10) according to one of the preceding claims.

5. Method for receiving at least one sample, in particular an organic substance, in a chamber (10), wherein the chamber (10) comprises a projection (12) in the region of an opening (11) of the chamber that is located at an upper side of the chamber (10) and wherein the projection (12) has a form of a tapering edge, comprising the following steps:
a) introducing a preferably bar-shaped sample carrier (15) into the chamber (10) and fixing the sample carrier (15) by at least one further projection (13) located in a lower region of the chamber (10) with respect to the upper side, wherein at least one of the projections (12, 13) is formed in one piece with at least part of the chamber (10),
b) moving the sample carrier (15) towards the projection (12) in the chamber (10) to exert a force applied by the projection (12) on the part of the received sample carrier (15) that extends into the chamber (10), so that a part (16) of the sample carrier is detached from a remainder (17) of the sample carrier (15).

6. Method according to Claim 6, wherein after steps a and b the following step is performed:
d) closing the opening (11) of the chamber (10).

## Revendications

1. Chambre (10) destinée à accueillir au moins un échantillon, notamment une substance organique, comprenant une ouverture (11) au niveau d'un côté supérieur de la chambre (10) servant à accueillir un porte-échantillon (15) de préférence en forme de tige, la chambre (10) possédant une partie saillante (12) dans la zone de l'ouverture (11), la partie saillante (12) étant conçue pour, par une forme d'un bord qui s'amincit, lors de l'action d'une force de la partie saillante (12) sur une partie du porte-échantillon (15) accueilli qui s'étend dans la chambre (10), séparer une partie (16) du porte-échantillon d'un reste (17) du porte-échantillon (15), **caractérisée en ce que** la chambre (10) possède au moins une partie saillante (13) supplémentaire dans une zone de la chambre (10) inférieure par rapport au côté supérieur, laquelle est conçue pour la fixation du porte-échantillon (15) accueilli, et au moins l'une des parties saillantes (12, 13) étant façonnée d'un seul tenant avec au moins une partie de la chambre (10).

2. Chambre (10) selon l'une des revendications précédentes, **caractérisée en ce que** l'ouverture (11) de la chambre (10) peut être fermée.

3. Chambre (10) selon l'une des revendications précédentes, **caractérisée en ce que** la chambre (10) fait partie d'un dispositif servant à la lyse des cellules, notamment à lyse au moyen d'ultrasons.

4. Arrangement pour l'analyse d'au moins un échantillon, notamment d'une substance organique, possédant une chambre (10) selon l'une des revendications précédentes.

5. Procédé pour accueillir au moins un échantillon, notamment une substance organique, dans une chambre (10), la chambre (10) comportant une partie saillante (12) dans la zone d'une ouverture (11) qui se trouve au niveau d'un côté supérieur de la chambre (10) et la partie saillante (12) possédant la forme d'un bord qui s'amincit, le procédé comprenant les étapes suivantes :
a) introduction d'un porte-échantillon (15) de préférence en forme de tige dans la chambre (10) et fixation du porte-échantillon (15) par au moins une partie saillante (13) supplémentaire qui se trouve dans une zone de la chambre (10) inférieure par rapport au côté supérieur, au moins l'une des parties saillantes (12, 13) étant façonnée d'un seul tenant avec au moins une partie de la chambre (10),
b) déplacement du porte-échantillon (15) contre la partie saillante (12) dans la chambre (10) en vue d'exercer une action de force de la partie saillante (12) sur la partie du porte-échantillon (15) accueilli qui s'étend dans la chambre (10), de sorte qu'une partie (16) du porte-échantillon est séparée d'un reste (17) du porte-échantillon (15).

6. Procédé selon la revendication 6, l'étape suivante étant exécutée après les étapes a et b :
d) fermeture de l'ouverture (11) de la chambre (10).
